Europäisches Patentamt

European Patent Office

Office européen des brevets

(11) Veröffentlichungsnummer : **0 345 248 B1**

(12) **EUROPÄISCHE PATENTSCHRIFT**

(45) Veröffentlichungstag der Patentschrift :
31.03.93 Patentblatt 93/13

(51) Int. Cl.⁵ : **A61K 31/095**, A61K 31/70,
A61K 31/195

(21) Anmeldenummer : 89890158.2

(22) Anmeldetag : 01.06.89

(54) **Verwendung des Enzym-Cofaktors NADPH zur Herstellung eines Arzneimittels sowie daraus hergestelltes Arzneimittel und Verfahren zu seiner Herstellung.**

(30) Priorität : 03.06.88 AT 1454/88

(43) Veröffentlichungstag der Anmeldung :
06.12.89 Patentblatt 89/49

(45) Bekanntmachung des Hinweises auf die
Patenterteilung :
31.03.93 Patentblatt 93/13

(84) Benannte Vertragsstaaten :
AT BE CH DE ES FR GB GR IT LI LU NL SE

(56) Entgegenhaltungen :
EP-A- 0 234 733
DE-A- 3 542 309
US-A- 4 579 870
Medline abs. 86256707 Cadet J.L. : " The potential use of vitamin E and selenium in parkinsonism. "

(56) Entgegenhaltungen :
Medline abs. 86319058 ANNEREN G. et al: "
Increased glutathione peroxid ase activity in
erythrocytes in patients with Alzheimer's dise-
ase/senile dementia of Alzheimer"
Medline abs. 85068581 CLAUSEN J. : " Demential syndromes and the lipid metabolism. "

(73) Patentinhaber : Birkmayer, Jörg, Prof.-Dr.
Schwarzspanierstrasse 15
A-1090 Wien (AT)

(72) Erfinder : Birkmayer, Jörg, Prof.-Dr.
Schwarzspanierstrasse 15
A-1090 Wien (AT)

(74) Vertreter : Haffner, Thomas M., Dr. et al
Patentanwaltskanzlei Dipl.-Ing. Adolf
Kretschmer Dr. Thomas M. Haffner
Schottengasse 3a
A-1014 Wien (AT)

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf die Verwendung des Enzym-Cofaktors NADPH oder eines Salzes davon zur Herstellung eines zur Behandlung des Parkinson-Syndroms geeigneten Arzneimittels, sowie ein Arzneimittel zur Behandlung des Parkinson-Syndroms enthaltend den Enzym-Cofaktor NADPH oder ein Salz hievon, und ein Verfahren zu seiner Herstellung.

Die Parkinson'sche Krankheit beruht auf einer gestörten dopaminergen Neurotransmission in den Basalganglien, im allgemeinen infolge eines zunehmenden Untergangs der dopaminergen Neuronen zunächst in der Substantia nigra, im Verlauf der Krankheit auch in anderen Arealen. In fortgeschrittenen Fällen wird auch ein zunehmender Noradrenalin-Mangel (z.B. im Locus caeruleus) feststellbar. Schon in frühen Stadien der Erkrankung findet man auch einen sehr deutlichen Abfall der Tyrosin-Hydroxylase-Aktivität, die interessanterweise auch in anderen Systemen und Organen (z.B. im Nebennierenmark) stark vermindert ist (siehe Tabelle 3 aus Birkmayer und Riederer, Die Parkinson-Krankheit, 2. Aufl., S. 34, Springer Verlag Wien, 1985).

Tabelle 3. *Tyrosinhydroxylase in verschiedenen Gehirnarealen beim Morbus Parkinson*

| Gehirnareale | | Kontrollen | M. Parkinson |
|---|---|---|---|
| N. caudatus | (15) | 27,8 ± 2,3 | 3,5 ± 1,0 (6)* |
| Putamen | (5) | 16,2 ± 5,9 | 1,2 ± 0,4 (6)* |
| S. nigra | (4) | 19,4 ± 6,2 | 4,9 ± 1,8 (4)* |
| L. caeruleus | (4) | 3,3 ± 0,1 | 2,0 ± 0,6 (2) |
| N. ruber | (5) | 5,7 ± 1,9 | 2,1 ± 1,4 (3) |
| Raphe + R.F. | (4) | 0,9 ± 0,6 | 1,5 ± 0,4 (5) |
| Hypothalamus | (5) | 3,1 ± 1,0 | 1,5 ± 0,3 (3) |
| C. mamillare | (5) | 0,6 ± 0,4 | 0,5 ± 0,9 (2) |
| N. accumbens | (5) | 2,0 ± 0,7 | 2,7 ± 2,2 (3) |
| Nebenniere (Medulla) | (5) | 186,2 ± 5,5 | 49,7 ± 12,4 (4)* |

Anzahl der Patienten in Klammern.
Mittelwerte ± sem (nmol Dopa/g-Gewebe. Stunde).
* $p < 0,01$.

Bei der konventionellen Therapie wird die fehlende oder verminderte dopaminerge Aktivität durch die Dopamin-Vorstufe L-DOPA ersetzt; bei Nachlassen dieser Wirkung kann die L-DOPA-Wirkung durch die spezifische Hemmung des Dopamin-abbauenden Enzyms MAO-B nochmals gesteigert werden.

Diese Behandlungsstrategien führten jedoch, obwohl durch sie die Lebenserwartung der Patienten verbessert wurde, nach Jahren der Anwendung häufig zu schweren und nicht vorhersagbaren Schwankungen der Beweglichkeit (Fluktuationen, on-off-Phänomene), die die Lebensqualität der Patienten empfindlich beeinträchtigen. Diese können teilweise durch rechtzeitigen Einsatz von postsynaptischen Agonisten, wie Lisurid, vermieden werden oder zumindest vermindert werden.

Für die plötzliche Einschränkung der Beweglichkeit wurde von Birkmayer und Riederer diskutiert, daß ihr eine vorübergehende Enzymerschöpfung der Tyrosin-Hydroxylase (Birkmayer und Riederer, Die Parkinson-Krankheit, 2. Aufl., S. 81, Springer Verlag Wien, 1985) zugrunde liegt. In der Tat läßt sich auch biochemisch durch einen Teil der Standard-Therapien eine vermehrte Belastung der Tyrosin-Hydroxylase postulieren.

Es ist anzunehmen, daß unter normalen Bedingungen dieses Schlüssel-Enzym der Dopamin- (und Noradrenalin-) Synthese ausreichend induziert und mit Co-Faktoren versorgt ist. Beim Parkinsonismus kann jedoch ein Fehler der Programmierung der Tyrosin-Hydroxylase-Synthese ursächlich beteiligt sein.

Klinische Untersuchungen haben überraschenderweise ergeben, daß auch bei Patienten mit fortgeschrittenem Parkinsonismus und schweren Schwankungen der Beweglichkeit die Gabe des Enzym-Cofaktors NADPH (Nikotinamid-Adenin-phosphatdinucleotid) in reduzierter Form eine geradezu dramatische Besserung der Motorik verursacht. Wesentlich war hierbei die Verwendung der reduzierten Form, da sich NADP als weitgehend wirkungslos erwies.

Die klinischen Ergebnisse sind am Beispiel von 2 Parkinson-Kranken in der nachfolgenden Tabelle dargestellt.

Fall 1: Krankheitsdauer 3 Jahre, Alter 43 Jahre, ohne Therapie

Erste Untersuchung 11.05.1987, seit einem Jahr Schmerzen in beiden Schultern und Zittern der linken Hand, Nachschleifen des linken Fußes. Objektiv: beim Gehen wird der linke Fuß nachgeschleift. Stoßen nach oben mit dem linken Arm nicht möglich bis zur vollen Streckung. Sprache leicht dysarthrisch. Mitbewegungen links fehlen.
Diagnose: Hemiparkinson, Disability 20.
Therapie: Madopar (R) 125 (= L-DOPA + Benserazid) 3-mal täglich Jumex (R) (= Deprenyl) 2-mal eine halbe Tablette.
Kontrolle 18.11.1987. Vorzeitige Ermüdung, Rigor der linken Körperhälte, Gang trippelnd, Schleifen des linken Fußes. Disability 40, abends off-Phase von 2-3 Stunden, Steifheit der linken Seite.
NADPH 25 mg 1-mal wöchentlich, kurzfristige Besserung,
Kontrolle 23.03.1988: Hemiparkinson links, Disability 50, off-Phase täglich 2-3 Stunden.
Therapie: NADPH 25 mg, 2-mal wöchentlich,
Kontrolle: 10.05.1988. Volle normale Bewegungsfähigkeit, weder Rigor noch Tremor, NADPH i.m. 25 mg, 1-mal wöchentlich garantiert 3-5 Tage eine normale Beweglichkeit. Beim Nachlassen der Wirkung genügt eine Madopar (R) 125 täglich. Fortsetzung der Therapie mit NADPH ohne Madopar (R). Disability 0, völlig normale Beweglichkeit, abends müde, Bewegungen verlangsamt.

Fall 2: Krankheitsdauer 3 Jahre, Alter 58 Jahre

Beim Schifahren Schädeltrauma, 4 Stunden bewußtlos (13.02.1985). Nach 3 Jahren Schleifen des rechten Fußes, leise Sprache, Springen unmöglich.
Diagnose: posttraumatischer Parkinson,
Therapie: Madopar (R) 200/50 (= L-DOPA + Benserazid) 3-mal eine halbe Tablette täglich, Jumex (R) (= Deprenyl) eine täglich.
Disability 30 (13.02.1985)
29.01.1986 Disability 15,
Zustand gebessert, Fortsetzung der Therapie.
Kontrolle: 23.02.1988.
Seit einem Jahr bewußt, daß seine Krankheit fortschreitet. Gang kleinschrittig, schlurfend, Stoß nach oben nicht möglich, Springen kommt nicht vom Boden, Sprache leise und schlecht artikuliert.
RR 100/80, im Stehen 90/70.
Therapie: Madopar (R) 125/6 (= L-DOPA + Benserazid) täglich, Jumex 2 täglich.
Disability 40.
14.4.1988 Disability 40,
Therapie: NADPH 25 mg, 2-mal wöchentlich i.m.
Disability nach einer Woche 20 (am Beginn der Wirkung kurzfristig Hyperkinesen. Unterbrechung der Madopar (R)-Behandlung, nach 3 Wochen Disability 15 (leichte Schwäche bei längerem Gehen). Nach Fortsetzung dieser Injektionen ist die Wirkungsdauer verlängert, nimmt 2 Madopar (R) 125 (= L-DOPA + Benserazid) täglich und 2-mal wöchentlich NADPH 25 mg.
Die Daten der Patienten zeigen, daß die Einnahme von NADPH oder eines Salzes davon zu einer deutlichen Verbesserung der Disability führt. Dies wird sowohl in der on- wie auch in der off-Phase festgestellt und wird sowohl bei gleichzeitiger Gabe von Standard-Präparation, wie auch ohne gleichzeitige Gabe von bekannten Parkinsonmitteln erreicht. Unter der erfindungsgemäßen Medikation wird auch eine Verkürzung der off-Phase erreicht.
Zur erfindungsgemäßen Verwendung kann das Co-Enzym NADPH oder sein physiologisch verträgliches Salz in üblicher Weise mit pharmazeutisch annehmbaren Hilfs- und Trägerstoffen konfektioniert werden. Gegebenenfalls kann NADPH auch in Kombination mit anderen Wirkstoffen, beispielsweise postsynaptischen Dopaminagonisten, wie Lisurid oder Amorphin, verwendet werden. Besonders wirkungsvoll erwies sich die gleichzeitige Verabreichung monosubstituierter selenorganischer Verbindungen, wie Selenaminosäuren, Selenproteine und Selenpolypeptide, wobei ein Synergismus festgestellt wurde, dessen Wirkung über die isolierte Darreichung von monosubstituierten selenorganischen Verbindungen und NADPH hinausging.
Zur Verwendung als Arzneimittel kann NADPH in die üblichen galenischen Zubereitungsformen für die orale, parenterale (wie intravenös oder subkutan) oder sublinguale Anwendung eingearbeitet werden. Die Präparate können in fester Form als Tabletten, Kapseln, Dragees oder in flüssiger Form als Lösungen, Suspensionen, Sprays oder Emulsionen sowie Zubereitungsformen mit verzögerter Wirkstoffgabe vorliegen. Zur Verbesserung der Motorik bei Parkinson-Patienten beträgt die Einzeldosis bei parenteraler Applikation zwischen 5 bis 500 mg, vorzugsweise zwischen 25 bis 100 mg und die Tagesdosis zwischen 5 bis 1500 mg, vorzugsweise 25 bis 300 mg.

Die oralen Dosen können im gleichen Bereich wie die parenteral anzuwendenden Wirkstoffmengen liegen oder gegebenenfalls um 1000% höher.

Liegt das Co-Enzym NADPH in Form seines physiologisch verträglichen Salzes vor, so sind hiefür alle bekannten sauren und basischen Salzbildner geeignet, wie organische und anorganische Säuren, wie z.B. Salzsäure, Schwefelsäure, Zitronensäure, Maleinsäure und Alkali- und Erdalkali-hydroxide und -salze.

## Patentansprüche

### Patentansprüche für folgende Vertragsstaaten : AT, BE, CH, DE, FR, GB, GR, IT, LI, LU, NL, SE

1. Verwendung des Enzym-Cofaktors NADPH oder eines Salzes davon zur Herstellung eines zur Behandlung des Parkinson-Syndroms geeigneten Arzneimittels.

2. Verwendung nach Anspruch 1, dadurch gekennzeichnet, daß zusätzlich zu NADPH monosubstituierte selenorganische Verbindungen gewählt aus der Gruppe Selenaminosäuren, Selenproteine und Selenpolypeptide, wie z.B. Selenmethionin, Selencystein, Selentyrosin od.dgl. verwendet werden.

3. Arzneimittel zur Behandlung des Parkinson-Syndroms enthaltend den Enzym-Cofaktor NADPH oder ein Salz hievon sowie gegebenenfalls monosubstituierte selenorganische Verbindungen.

4. Arzneimittel nach Anspruch 3, dadurch gekennzeichnet, daß NADPH in Mengen von 5 bis 500 mg, vorzugsweise 25 bis 100 mg, je Einheit der Darreichungsform enthalten ist.

5. Verfahren zur Herstellung eines Arzneimittels gemäß Anspruch 3 oder 4, dadurch gekennzeichnet, daß man den Enzym-Cofaktor NADPH oder ein Salz hievon sowie gegebenenfalls monosubstituierte selenorganische Verbindungen, wie Selenaminosäuren, Selenproteine und Selenpolypeptide mit geeigneten Träger-, Hilfs- und/oder Zusatzstoffen in eine geeignete Darreichungsform bringt.

### Patentansprüche für folgenden Vertragsstaat : ES

1. Verfahren zur Herstellung eines Arzneimittels zur Behandlung des Parkinson-Syndroms enthaltend den Enzym-Cofaktor NADPH oder ein Salz hievon sowie gegebenenfalls monosubstituierte selenorganische Verbindungen, dadurch gekennzeichnet, dass man den Enzym-Cofaktor NADPH oder ein Salz hievon sowie gegebenenfalls monosubstituierte selenorganische Verbindungen, wie Selenaminosäuren, Selenproteine und Selenpolypeptide, wie z.B. Selenmethionin, Selencystein, Selentyrosin od. dgl. mit geeigneten Träger-, Hilfs- und/oder Zusatzstoffen in eine geeignete Darreichungsform bringt.

2. Verfahren nach Anspruch 1, dadurch gekennzeichnet, dass NADPH in Mengen von 5 bis 500 mg, vorzugsweise 25 bis 100 mg, je Einheit der Darreichungsform eingesetzt wird.

## Claims

### Claims for the following Contracting States : AT, BE, CH, DE, FR, GB, GR, IT, LI, LU, NL, SE

1. Use of the enzyme-cofactor NADPH or a salt thereof for the preparation of a pharmaceutical suitable for the treatment of Parkinson's syndrome.

2. Use in accordance with Claim 1, characterised in that monosubstitute selenium compounds selected from the group of selenium amino acids, selenium proteins and selenium polypeptides, such as selenium methionine, selenium cysteine, selenium tyrosine, or the like, are used in addition to NADPH.

3. Pharmaceutical for the treatment of Parkinson's syndrome containing the enzyme-cofactor NADPH or a salt thereof, together with monosubstitute selenium compounds, where appropriate.

4. Pharmaceutical in accordance with Claim 3, characterised in that NADPH is contained in quantities of between 5 and 500 mg, preferably between 25 and 100 mg, per unit of the presentation form.

5. Procedure for the preparation of a pharmaceutical in accordance with Claims 3 or 4, characterised in that the enzyme-cofactor NADPH or a salt thereof and, where appropriate, monosubstitute selenium compounds, such as selenium amino acids, selenium proteins and selenium polypeptides, are combined with appropriate excipient, vehicle and/or adjuvant substances in a suitable presentation form.

**Claims for the following Contracting State : ES**

1. Process for the preparation of a pharmaceutical for the treatment of Parkinson's syndrome containing the enzyme-cofactor NADPH or a salt thereof and optionally monosubstituted selenium-organic compounds, characterised in that the enzyme-cofactor NADPH or a salt thereof and where appropriate monosubstituted selenium-organic compounds, such as selenium amino acids, selenium proteins and selenium polypeptides such as selenium methionine, selenium cysteine, selenium tyrosine or the like are brought in a suitable presentation form together with appropriate excipients, vehicles and/or adjuvants.

2. Process according to claim 1, characterised in that NADPH is used in quantities of between 5 and 500 mg, preferably between 25 and 100 mg per unit of the presentation form.

**Revendications**

**Revendications pour les Etats contractants suivants : AT, BE, CH, DE, FR, GB, GR, IT, LI, LU, NL, SE**

1. Application du cofacteur d'enzyme NADPH ou d'un de ses sels à la préparation d'un médicament approprié au traitement de la maladie de Parkinson.

2. Application selon la revendication 1, caractérisée en ce qu'on utilise, outre le NADPH, des composés sélénio-organiques mono-substitués choisis dans le groupe des acides sélénio-aminés, des sélénio-protéines et des sélénio-polypeptides, comme p. ex. la sélénio-méthionine, la sélénio-cystéine, la sélénio-tyrosine, etc.

3. Médicament pour le traitement du syndrome de Parkinson contenant le cofacteur d'enzyme NADPH ou un de ses sels ainsi que des composés sélénio-organiques éventuellement mono-substitués.

4. Médicament selon la revendication 3, caractérisé en ce qu'il contient du NADPH à des quantités de 5 à 500 mg, de préférence 25 à 100 mg, par unité de forme d'administration.

5. Procédé de préparation d'un médicament selon la revendication 3 ou 4, caractérisé en ce qu'on met sous une forme d'administration appropriée le cofacteur d'enzyme NADPH ou un de ses sels ainsi que des composés sélénio-organiques éventuellement monosubstitués, comme les acides sélénio-aminés, les sélénio-protéines et les sélénio-polypeptides avec des supports, adjuvants et/ou additifs appropriés.

**Revendications pour l'Etat contractant suivant : ES**

1. Procédé de préparation d'un médicament approprié au traitement de la maladie de Parkinson contenant le cofacteur d'enzyme NADPH ou d'un de ses sels et des composés sélénio-organiques éventuellement mono-substitués caractérisée en ce qu'on met sous une forme d'administration appropriée le cofacteur d'enzyme NADPH ou un de ses sels ainsi que des composés sélénio-organiques éventuellement mono-substitués, comme les acides sélénio-aminés, les sélénio-protéines et les sélénio-polypeptides avec des supports, adjuvants et/ou additifs appropriés.

2. Procédé selon la revendication 1, characterisée en ce que le NADPH est contenu à des quantités de 5 à 500 mg, de préférence 25 à 100 mg, par unité de forme d'administration.